(19) Europäisches Patentamt
European Patent Office
Office européen des brevets

(11) **EP 3 342 339 A1**

(12) **EUROPEAN PATENT APPLICATION**
published in accordance with Art. 153(4) EPC

(43) Date of publication:
**04.07.2018 Bulletin 2018/27**

(51) Int Cl.:
*A61B 5/055* (2006.01)     *A61B 5/00* (2006.01)
*G01R 33/54* (2006.01)     *G01R 33/56* (2006.01)

(21) Application number: **16875872.0**

(22) Date of filing: **08.08.2016**

(86) International application number:
**PCT/KR2016/008670**

(87) International publication number:
**WO 2017/104930 (22.06.2017 Gazette 2017/25)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **17.12.2015 KR 20150181080**

(71) Applicant: **Samsung Electronics Co., Ltd.**
**Suwon-si, Gyeonggi-do 16677 (KR)**

(72) Inventors:
• **SONG, Myung-sung**
**Hwaseong-si**
**Gyeonggi-do 18445 (KR)**
• **CHOI, Joon-sung**
**Anyang-si**
**Gyeonggi-do 14078 (KR)**

(74) Representative: **D'Halleweyn, Nele Veerle Trees Gertrudis et al**
**Arnold & Siedsma**
**Bezuidenhoutseweg 57**
**2594 AC The Hague (NL)**

(54) **MAGNETIC RESONANCE IMAGING APPARATUS AND METHOD THEREFOR**

(57)     Provided are a magnetic resonance imaging (MRI) apparatus and a method of processing an MR image. The MRI apparatus includes an MR signal receiver receiving an MR signal emitted from the object, and a processor configured to obtain k-space blades from the MR signal according to a periodically rotated overlapping parallel lines with enhanced reconstruction (PROPELLER) method, to obtain centroid data of the k-space blades, and to determine whether to reconstruct an MR image of the object.

FIG. 1

EP 3 342 339 A1

**Description**

TECHNICAL FIELD

The present disclosure relates to magnetic resonance imaging (MRI) apparatuses and methods therefor. More particularly, the present disclosure relates to MRI apparatuses and methods of capturing magnetic resonance (MR) images by using a periodically rotated overlapping parallel lines with enhanced reconstruction (PROPELLER) method.

BACKGROUND ART

A magnetic resonance imaging (MRI) apparatus is an apparatus for imaging an object by using a magnetic field, and is widely used to accurately diagnose a disease because the MRI apparatus is capable of three-dimensionally displaying discs, joints, nerves, ligaments, and heart, as well as bones, from a desired angle.

When a magnetic resonance (MR) image is captured, the MRI apparatus irradiates a radio frequency (RF) signal to an object and obtains an MR signal emitted from the object according to the RF signal. In order to obtain MR images of high image quality, the MRI apparatus may reconstruct an MR image, in which motion of the object during the capturing of the magnetic resonance image is corrected, by using the MR signal.

When the motion of the object generated while the MR image is captured exceeds a correctable range, it is necessary to obtain the MR signal again. In order to determine whether the motion of the object exceeds the correctable range, the MRI apparatus may determine whether the obtained MR signal is within a motion-correctable range after reconstructing the MR image. When it is determined that the motion-correctable range has been exceeded through the reconstructed MR image, the MRI apparatus has to re-capture an MR image in order to obtain an MR signal again. As such, when the motion of the object exceeds a correctable range, a total time taken to capture the MR image increases.

Therefore, there is a need for a method of determining in advance whether the motion of the object generated during the capturing of the MR image is within the correctable range, before reconstructing the image.

DESCRIPTION OF EMBODIMENTS

TECHNICAL PROBLEM

According to an aspect of the present disclosure, after an MR image is captured by using a periodically rotated overlapping parallel lines with enhanced reconstruction (PROPELLER) method, it may be determined whether motion of an object represented by a magnetic resonance (MR) signal is within a correctable range of the MR image before reconstructing the MR image.

According to an aspect of the present disclosure, after capturing an MR image by using the PROPELLER method, when there is data of an obtained MR signal, the data exceeding a motion-correctable range, the data may only be re-obtained to reduce a time taken to re-perform image capture.

SOLUTION TO PROBLEM

In accordance with an aspect of the present disclosure, a magnetic resonance imaging (MRI) apparatus includes: a magnetic resonance (MR) signal receiver configured to obtain an MR signal emitted from an object; and a processor configured to obtain k-space blades from the MR signal by a periodically rotated overlapping parallel lines with enhanced reconstruction (PROPELLER) method, to obtain centroid data of the k-space blades, and to determine whether to reconstruct an MR image of the object based on the centroid data.

The processor may be configured to reconstruct the MR image of the object based on whether there is a piece of centroid data exceeding an error range.

When there is a piece of centroid data exceeding the error range among the centroid data, the processor may be configured to reobtain a k-space blade having the piece of centroid data exceeding the error range.

When there is no centroid data exceeding the error range, the processor may be configured to determine that the MR image is to be reconstructed from the k-space blades.

The MRI apparatus may further include a display configured to display a graph illustrating magnitudes of the centroid data and illustrating whether the magnitudes of the centroid data exceed the error range.

The MRI apparatus may further include a display configured to display a user interface including MR images corresponding to the k-space blades.

The MRI apparatus may further include an input unit configured to receive an input about whether to reobtain the k-space blades.

Each of the k-space blades may be obtained by one scanning operation.

The processor may be configured to reconstruct an MR image, in which a motion is corrected, by using the k-space blades.

The MRI apparatus may further include a display configured to display the MR image that is reconstructed.

In accordance with an aspect of the present disclosure, a method for a magnetic resonance imaging (MRI) apparatus, the method includes: obtaining a magnetic resonance (MR) signal emitted from an object; obtaining k-space blades from the MR signal according to a periodically rotated overlapping parallel lines with enhanced reconstruction (PROPELLER) method; obtaining centroid data of the k-space blades; and determining whether to reconstruct an MR image of the object based on the centroid data.

The determining of whether to reconstruct the MR image may include performing the determination based on whether there is a piece of centroid data exceeding an error range, from among the centroid data of the k-space blades.

The method may further include, when there is a piece of centroid data exceeding the error range among the centroid data, reobtaining a k-space blade having the piece of centroid data exceeding the error range.

The method may further include, when there is no centroid data exceeding the error range, determining that an MR image is to be reconstructed from the k-space blades.

The method may further include displaying a graph illustrating the centroid data and illustrating whether each piece of the centroid data exceeds the error range.

The method may further include displaying a user interface including an MR image corresponding to the k-space blades.

The method may further include receiving an input regarding whether to reobtain the k-space blades.

Each of the k-space blades may be obtained by one scanning operation.

The method may further include reconstructing an MR image in which a motion is corrected, by using the k-space blades.

The method may further include displaying the MR image that is reconstructed.

In accordance with an aspect of the present disclosure, there is provided a non-transitory computer-readable recording medium having recorded thereon a program, which when executed by a computer, performs the method for the MRI apparatus.

ADVANTAGEOUS EFFECTS OF DISCLOSURE

According to the present disclosure, after capturing a magnetic resonance (MR) image by using a periodically rotated overlapping parallel lines with enhanced reconstruction (PROPELLER) method, it may be determined whether the motion of object shown in the MR image is within the correctable range before reconstructing the image.

Also, according to the present disclosure, after capturing the MR image by using the PROPELLER method, when there is data, in an obtained MR signal, exceeding the motion-correctable range, the data only is obtained again, and thus, a time taken to re-perform image capture may be reduced.

BRIEF DESCRIPTION OF THE DRAWINGS

FIG. 1 is a diagram illustrating a magnetic resonance imaging (MRI) apparatus according to an embodiment, the MRI apparatus obtaining a magnetic resonance image of an object by using a periodically rotated overlapping parallel lines with enhanced reconstruction (PROPELLER) method.

FIG. 2 is a diagram illustrating an MRI apparatus according to an embodiment, the MRI apparatus obtaining a magnetic resonance image of an object by using a PROPELLER method.

FIG. 3 is a diagram of an MRI apparatus according to an embodiment of the present disclosure.

FIG. 4 is a graph displayed by an MRI apparatus, according to an embodiment of the present disclosure.

FIG. 5 is a flowchart illustrating a method of processing a magnetic resonance (MR) image, according to an embodiment of the present disclosure.

FIG. 6 is a flowchart illustrating a method of processing an MR image, according to an embodiment of the present disclosure.

FIG. 7A is a graph displayed by an MRI apparatus, according to an embodiment of the present disclosure.

FIG. 7B is a diagram of a user interface displayed by an MRI apparatus, according to an embodiment of the present disclosure.

FIG. 7C is a diagram of a user interface displayed by an MRI apparatus, according to an embodiment of the present disclosure.

FIG. 8 is a schematic diagram of an MRI system.

FIG. 9 is a diagram of a communicator according to an embodiment of the present disclosure.

MODE OF DISCLOSURE

Advantages and features of one or more embodiments of the present invention and methods of accomplishing the same may be understood more readily by reference to the following detailed description of the embodiments and the accompanying drawings. In this regard, the present embodiments may have different forms and should not be construed as being limited to the descriptions set forth herein. Rather, these embodiments are provided so that this disclosure will be thorough and complete and will fully convey the concept of the present embodiments to one of ordinary skill in the art, and the present invention will only be defined by the appended claims.

Terms used herein will now be briefly described and then one or more embodiments of the present invention will be described in detail.

All terms including descriptive or technical terms which are used herein should be construed as having meanings that are obvious to one of ordinary skill in the art. However, the terms may have different meanings according to the intention of one of ordinary skill in the art, precedent cases, or the appearance of new technologies. Also, some terms may be arbitrarily selected by the applicant, and in this case, the meaning of the selected terms will be described in detail in the detailed description of the invention. Thus, the terms used herein have to be defined based on the meaning of the terms together with the description throughout the specification.

When a part "includes" or "comprises" an element, unless there is a particular description contrary thereto, the part can further include other elements, not excluding the other elements. Also, the term "unit" in the embodiments of the present invention means a software component or hardware component such as a field-programmable gate array (FPGA) or an application-specific integrated circuit (ASIC), and performs a specific function. However, the term "unit" is not limited to software or hardware. The "unit" may be formed so as to be in an addressable storage medium, or may be formed so as to operate one or more processors. Thus, for example, the term "unit" may refer to components such as software components, object-oriented software components, class components, and task components, and may include processes, functions, attributes, procedures, subroutines, segments of program code, drivers, firmware, micro codes, circuits, data, a database, data structures, tables, arrays, or variables. A function provided by the components and "units" may be associated with the smaller number of components and "units", or may be divided into additional components and "units".

Reference will now be made in detail to embodiments, examples of which are illustrated in the accompanying drawings. In this regard, the present embodiments may have different forms and should not be construed as being limited to the descriptions set forth herein. In the following description, well-known functions or constructions are not described in detail so as not to obscure the embodiments with unnecessary detail.

In the present specification, an "image" may refer to multi-dimensional data composed of discrete image elements (e.g., pixels in a two-dimensional (2D) image and voxels in a three-dimensional (3D) image). For example, the image may be a medical image of an object captured by an X-ray apparatus, a computed tomography (CT) apparatus, a magnetic resonance imaging (MRI) apparatus, an ultrasound diagnosis apparatus, or another medical imaging apparatus.

Furthermore, in the present specification, an "object" may be a human, an animal, or a part of a human or animal. For example, the object may be an organ (e.g., the liver, the heart, the womb, the brain, a breast, or the abdomen), a blood vessel, or a combination thereof. Furthermore, the "object" may be a phantom. The phantom means a material having a density, an effective atomic number, and a volume that are approximately the same as those of an organism. For example, the phantom may be a spherical phantom having properties similar to the human body.

Furthermore, in the present specification, a "user" may be, but is not limited to, a medical expert, such as a medical doctor, a nurse, a medical laboratory technologist, and a technician who repairs a medical apparatus.

Furthermore, in the present specification, an "MR image" refers to an image of an object obtained by using the nuclear magnetic resonance principle.

Furthermore, in the present specification, a "pulse sequence" refers to continuity of signals repeatedly applied by an MRI apparatus. The pulse sequence may include a time parameter of a radio frequency (RF) pulse, for example, repetition time (TR) or echo time (TE).

Furthermore, in the present specification, a "pulse sequence schematic diagram" shows an order of events that occur in an MRI apparatus. For example, the pulse sequence schematic diagram may be a diagram showing an RF pulse, a gradient magnetic field, an MR signal, or the like according to time.

An MRI system is an apparatus for acquiring a sectional image of a part of an object by expressing, in a contrast comparison, a strength of a MR signal with respect to a radio frequency (RF) signal generated in a magnetic field having a specific strength. For example, if an RF signal that only resonates a specific atomic nucleus (for example, a hydrogen atomic nucleus) is emitted for an instant toward the object placed in a strong magnetic field and then such emission stops, an MR signal is emitted from the specific atomic nucleus, and thus the MRI system may receive the MR signal and acquire an MR image. The MR signal denotes an RF signal emitted from the object. An intensity of the MR signal may be determined according to a density of a predetermined atom (for example, hydrogen) of the object, a relaxation time T1, a relaxation time T2, and a flow of blood or the like.

MRI systems include characteristics different from those of other imaging apparatuses. Unlike imaging apparatuses such as CT apparatuses that acquire images according to a direction of detection hardware, MRI systems may acquire 2D images or 3D volume images that are oriented toward an optional point. MRI systems do not expose objects or examiners to radiation, unlike CT apparatuses, X-ray apparatuses, position emission tomography (PET) apparatuses, and single photon emission CT (SPECT) apparatuses, may acquire images having high soft tissue contrast, and may acquire neurological images, intravascular images, musculoskeletal images, and oncologic images that are required to precisely capturing abnormal tissues.

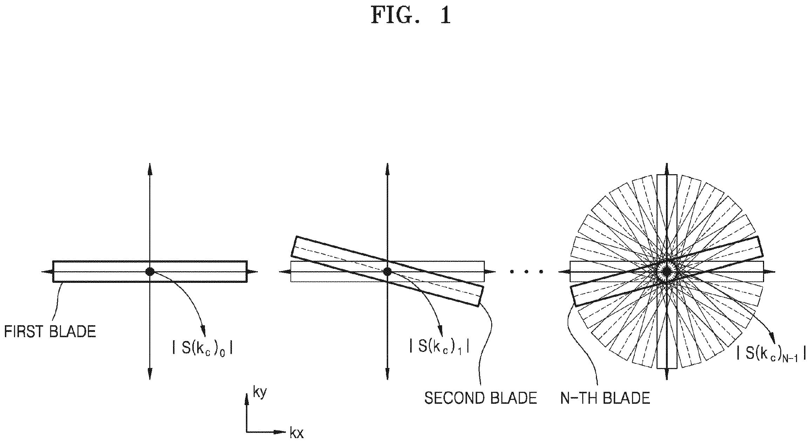

FIG. 1 is a diagram illustrating a magnetic resonance imaging (MRI) apparatus according to an embodiment, the MRI apparatus obtaining a magnetic resonance (MR) image of an object by using a periodically rotated overlapping parallel lines with enhanced reconstruction (PROPELLER) method.

An MR image may be obtained from an MR signal emitted from an object. The MR signal may be represented as k-space data corresponding thereto, and the k-space data may be reconstructed as an MR image via fast Fourier transform (FFT).

As shown in FIG. 1, an MR image representing a cross-section of an object may be obtained when the MRI apparatus obtains N k-space blades in a k-space according to the PROPELLER method.

In detail, when N k-space blades are obtained by the PROPELLER method, the k-space data may be obtained. Also, the k-space data obtained from the N blades may be reconstructed as an MR image. The reconstructed MR image may be an image representing the cross-section of the object.

Here, the k-space blade may denote a set of k-space data including a centroid of the k-space obtained according to the PROPELLER method. As shown in FIG. 1, each of the N blades may include k-space data of a portion surrounded by a square.

Each of the k-space blades may be arranged by rotation, by a predetermined angle, about a centroid of the k-space, that is, a point having a coordinate of an x-axis and a y-axis (0,0) in the k-space. For example, a second blade is obtained by rotating a first blade about the centroid of the k-space by a predetermined angle.

Each of the k-space blades may be obtained through one scan (or shot). That is, one k-space blade may be obtained by one pulse sequence. For example, when the MRI apparatus obtains N k-space blades, N-times of scanning operations are necessary. As described above, the MRI apparatus may obtain the k-space data through a plurality of scanning operations according to a multiple-shot fast spin-echo (FSE) method according to the related art.

As shown in FIG. 1, when the MRI apparatus obtains all of N blades including a first blade to an N-th blade, one MR image may be reconstructed based on the k-space data included in the N k-space blades.

As shown in FIG. 1, the first blade to the N-th blade may each include k-space data having a coordinate of an x-axis and a y-axis (0,0) in the k-space. Hereinafter, the k-space data having the coordinate of the x-axis and the y-axis (0,0) in the k-space will be referred to as centroid data or $S(k_c)$.

As shown in FIG. 1, when the MRI apparatus obtains N k-space blades, the first blade includes $S(k_c)_0$, the second blade includes $S(k_c)_1$, and the N-th blade includes $S(k_c)_{N-1}$.

When N k-space blades are obtained through N-times of scanning operations according to the PROPELLOR method, values of the centroid data may be consistent while the N-times of scanning operations are performed in a case where there is no motion of an object.

Therefore, when the object does not move while capturing the MR images, values of $S(k_c)$ are equal to one another as described below.

$$|S(k_c)_0| = |S(k_c)_1| = \ldots = |S(k_c)_{N-1}| \qquad \text{----------------} \ (1)$$

Here, the value of $S(k_c)$ may be related to an intensity of the MR signal. When there is a motion of the object during the capturing of the MR image, the intensity of the obtained MR signal varies, and accordingly, values of $S(k_c)$ may vary according to the N scanning operations.

For example, MR image data in a time domain may be referred to as $s(r)$, and data represented in the k-space may be referred to as $S(k)$. Here, MR image data moved by $r_0$ in the time domain may be referred to as $s(r-r_0)$, and the k-space data corresponding to $s(r-r_0)$ may be changed to $\exp(-j2\pi kr_0) \cdot S(k)$ according to characteristics of the Fourier transform as follows.

$$F\{s(r-r_0)\} = \exp(-j2\pi kr_0) \cdot S(k) \qquad \text{----------------} \ (2)$$

That is, when the object moves as much as $r_0$, the value of $S(k_c)$ may be equal to a value of $S(k_c)$ multiplied

by $exp(-j2\pi kr_0)$.

In addition, the motion of the object may be classified as a motion in parallel with an obtained cross-section (in-plane motion) and a motion in a direction perpendicular to the obtained cross-section (through-plane motion). In particular, when the through-plane motion of the object is generated, a correlation weight may be applied to correct the motion.

It may take a long time period to correct the through-plane motion of the object in the above manner. Also, when the motion of the object is relatively large, blur may occur in a reconstructed image even if the motion correction is performed.

Therefore, in order to reconstruct an MR image of high image quality, when the blur occurs in the reconstructed image, there may be a case in which the capturing of the image has to be performed again to include the motion of the object within a correctable range, according to the related art.

According to an embodiment of the present disclosure, whether to reconstruct the MR image of the object may be determined in advance by comparing the values of $S(k_c)$ obtained while obtaining the N blades.

In detail, according to the embodiment of the present disclosure, a degree of the motion of the object shown in reconstruction of the MR image may be determined in real time through the values of $S(k_c)$ obtained while obtaining the N blades.

Also, according to the embodiment of the present disclosure, it may be identified in real time whether the motion of the object is within an allowable range for reconstruction of the MR image through the values of $S(k_c)$ obtained in real time.

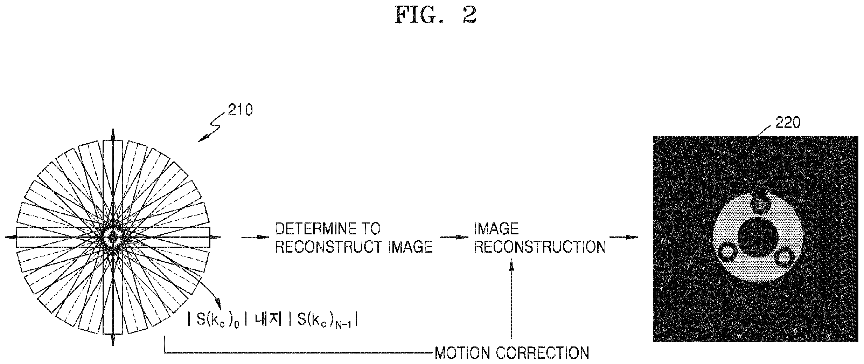

FIG. 2 is a diagram illustrating an MRI apparatus according to an embodiment obtaining an MR image of an object by using the PROPELLER method.

Referring to FIG. 2, the MRI apparatus according to the embodiment may obtain k-space blades 210 from the MR signal emitted from the object, by using a PROPELLER method.

In addition, the MRI apparatus according to the embodiment may obtain centroid data from the obtained k-space blades 210, and may obtain magnitude values of the centroid data, that is, $|S(k_c)_0|$ to $|S(k_c)_{N-1}|$.

The MRI apparatus according to the embodiment may determine whether to reconstruct the MR image of the object based on the magnitude values of the centroid data. In detail, the MRI apparatus according to the embodiment may determine whether to reconstruct the MR image of the object based on whether there is a piece of centroid data exceeding an error range. Here, the error range may denote a range of the centroid data, in which k-space data included in each k-space blade indicates that the motion correction of the object is possible.

In addition, when the MRI apparatus according to the embodiment determines that the MR image of the object should be reconstructed, a reconstructed image 220 may be obtained based on the k-space blades 210. The reconstructed image 220 may be an image in which the motion is corrected based on the k-space blades 210.

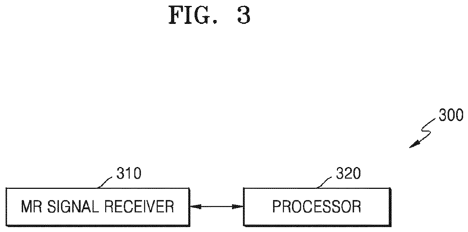

FIG. 3 is a diagram of an MRI apparatus 300 according to an embodiment of the present disclosure.

The MRI apparatus 300 of FIG. 3 may be an apparatus for processing an MR signal obtained by performing an MRI operation on the object.

The MRI apparatus 300 may include an MR signal receiver 310 and a processor 320.

The MR signal receiver 310 may obtain an MR signal emitted from the object. The MR signal receiver 310 may include a radio frequency (RF) coil, and may receive the MR signal emitted from the object by using the RF coil. The MR signal may be obtained by a multiple-shot fast spin-echo (FSE) method.

The processor 320 may reconstruct the MR image of the object by using the MR signal emitted from the object.

According to an embodiment, the processor 320 may obtain the k-space blades from the MR signal by the PROPELLER method. For example, the processor 320 may obtain N blades based on the MR signal that is obtained by the multiple-shot FSE method.

The processor 320 may obtain centroid data from the k-space blades, and may determine whether to reconstruct the MR image of the object based on the centroid data. Each of the k-space blades may be obtained by one scanning operation.

In detail, the processor 320 may determine whether to reconstruct the MR image of the object based on whether there is a piece of centroid data exceeding an error range.

When there is the piece of centroid data exceeding the error range, the processor 320 may reobtain the k-space blade having the piece of centroid data exceeding the error range.

When there is no piece of centroid data exceeding the error range, the processor 320 may determine that the MR image should be reconstructed from the k-space blades. Also, the processor 320 may reconstruct the MR image, in which the motion is corrected, by using the obtained k-space blades.

Also, the processor 320 may generate a graph illustrating the centroid data and illustrating whether each piece of the centroid data exceeds the error range.

In addition, the processor 320 may generate a user interface including an MR image corresponding to the k-space blades.

According to the MRI apparatus 300 of the embodiment, whether to reconstruct the MR image of the object may be determined in advance by comparing the values of $S(k_c)$ obtained while obtaining the N blades.

Also, according to the MRI apparatus 300, a degree of the object motion to be represented when reconstructing the MR image and whether to reconstruct the MR image of the object may be determined in real time through the values of $S(k_c)$ obtained in real time while obtaining the N blades.

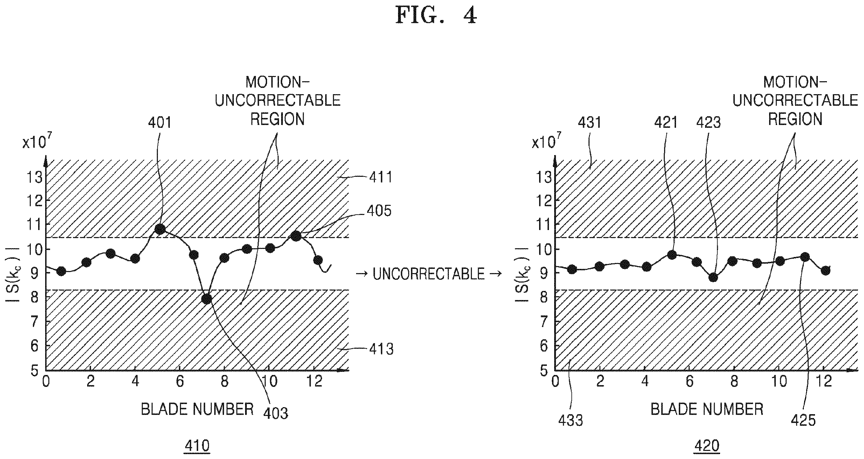

FIG. 4 is a graph displayed by the MRI apparatus 300 according to the embodiment of the present disclosure.

Referring to FIG. 4, the MRI apparatus 300 according to the embodiment may display graphs 410 and 420 illustrating the centroid data and whether each piece of the centroid data exceeds the error range.

The MRI apparatus 300 may display the graphs 410 and 420, in which a horizontal axis represents numbers of k-space blades and the vertical axis represents magnitude of the value of S(kc).

Referring to FIG. 4, the MRI apparatus 300 may display, for example, the graphs 410 and 420 illustrating magnitudes of values of $S(k_c)$ for twelve blades.

The MRI apparatus 300 may represent a motion-uncorrectable region in the graphs. The motion-uncorrectable region may be a region for representing the centroid data that exceeds the error range.

Referring to the graph 410 of FIG. 4, the MRI apparatus 300 may display a magnitude 401 of $S(k_c)$ of a fifth blade and a magnitude 405 of $S(k_c)$ of an eleventh blade in a motion-uncorrectable region 411. Also, the MRI apparatus 300 may display a magnitude 403 of $S(k_c)$ of a seventh blade in a motion-uncorrectable region 413.

The MRI apparatus 300 may determine whether to reconstruct the MR image of the object based on whether there is a piece of centroid data exceeding an error range.

For example, the MRI apparatus 300 may determine that there are pieces of centroid data exceeding the error range, based on the magnitude 401 of $S(k_c)$ of the fifth blade, the magnitude 403 of $S(k_c)$ of the seventh blade, and the magnitude 405 of $S(k_c)$ of the eleventh blade.

When there are pieces of centroid data exceeding the error range, the MRI apparatus 300 may reobtain the k-space blades having the pieces of centroid data exceeding the error range. For example, the MRI apparatus 300 may reobtain the fifth blade, the seventh blade, and the eleventh blade.

The graph 420 of FIG. 4 may be a graph that is updated after reobtaining the fifth blade, the seventh blade, and the eleventh blade. Referring to the graph 420 of FIG. 4, a magnitude 421 of $S(k_c)$ of the updated fifth blade, a magnitude 423 of $S(k_c)$ of the updated seventh blade, and a magnitude 425 of $S(k_c)$ of the updated eleventh blade are shown.

Referring to the graph 420 of FIG. 4, a magnitude 421 of $S(k_c)$ of the updated fifth blade, a magnitude 423 of $S(k_c)$ of the updated seventh blade, and a magnitude 425 of $S(k_c)$ of the updated eleventh blade may not be included in motion-uncorrectable regions 431 and 433.

When there is no centroid data exceeding the error range, the MRI apparatus 300 may determine that the MR image should be reconstructed from the k-space blades.

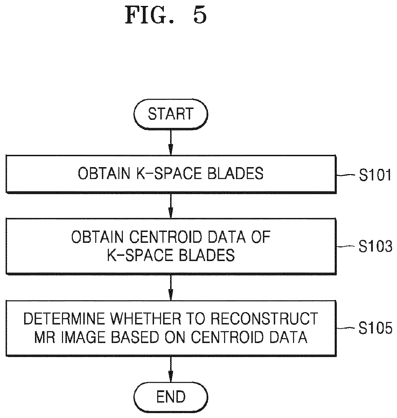

FIG. 5 is a flowchart illustrating a method of processing an MR image, according to an embodiment of the present disclosure.

In operation S101, the MRI apparatus 300 may obtain an MR signal emitted from an object (S101).

**In** operation S103, the MRI apparatus 300 may obtain centroid data of k-space blades (S103).

In detail, the MRI apparatus 300 may obtain the k-space blades from the MR signal by using the PROPELLER method, and may obtain the centroid data of the k-space blades.

Here, a k-space blade may denote a set of k-space data including a center point of a k-space obtained according to the PROPELLER method. Also, the centroid data may denote k-space data having a coordinate of the x-axis and the y-axis (0,0) in the k-space.

In operation S105, the MRI apparatus 300 may determine whether to reconstruct the MR image of the object based on the centroid data (S105).

For example, when N k-space blades are obtained by N scanning operations performed by the MRI apparatus 300 in the PROPELLER method, a variation in values of the centroid data may not be large during the N scanning operations when the motion of the object is not large.

That is, the MRI apparatus 300 may determine whether to reconstruct the MR image of the object based on the variation in the values of the centroid data of the k-space blades.

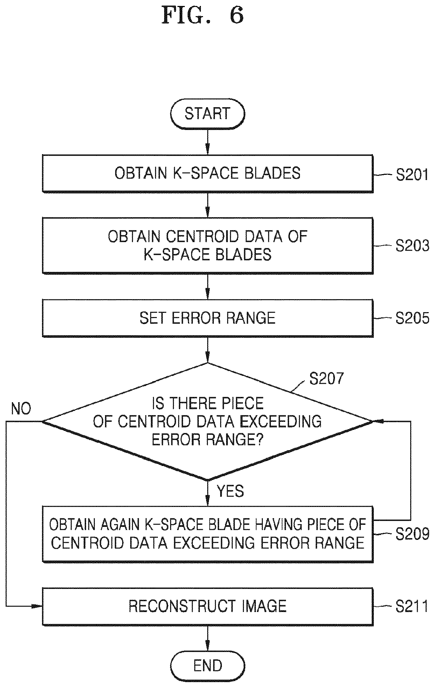

FIG. 6 is a flowchart illustrating a method of processing an MR image, according to an embodiment of the present disclosure.

In operation S201, the MRI apparatus 300 may obtain an MR signal emitted from an object (S201).

In operation S203, the MRI apparatus 300 may obtain centroid data of k-space blades (S203).

In detail, the MRI apparatus 300 may obtain the k-space blades from the MR signal by using the PROPELLER method, and may obtain the centroid data of the k-space blades.

In operation S205, the MRI apparatus 300 may set an error range (S205). The error range denotes a range of the centroid data, in which the k-space data included in the k-space blade indicates that the motion of the object is correctable. The error range may vary depending on characteristics of the image and the imaging environment. The error range may be automatically set for every imaging environment of the MRI apparatus 300, or the MRI apparatus 300 may receive an input for the error range.

In operation S207, the MRI apparatus 300 may determine whether there is a piece of centroid data exceeding the error range (S207). For example, the MRI apparatus 300 may determine whether there is a piece of centroid data exceeding the error range, based on whether the piece of centroid data is included in the motion-uncorrectable region.

When the MRI apparatus 300 determines that there is a piece of centroid data exceeding the error range, in operation S209, the MRI apparatus 300 may reobtain the k-space blade having the centroid data that exceeds the error range (S209).

After that, the MRI apparatus 300 may determine again whether there is a piece of centroid data exceeding the error range, in operation S207.

When the MRI apparatus 300 determines that there is no centroid data exceeding the error range, the MRI apparatus 300 may determine that the image should be reconstructed in operation S211. By using the k-space blades, the MRI apparatus 300 may reconstruct an MR image in which the motion is corrected.

According to the MRI apparatus 300 of the embodiment, when there is a k-space blade exceeding the motion-correctable range, from among the obtained k-space blades, after capturing the MR image by using the PROPELLER method, the k-space blade may only be reobtained to reduce the time taken to reperform the image capture.

In addition, according to the embodiment, even when the MRI apparatus 300 repeatedly reobtains the k-space blade having the centroid data that exceeds the error range, the centroid data exceeding the error range may still remain.

When there is a piece of centroid data exceeding the error range even if the k-space blade is repeatedly reobtained a predetermined number of times or greater, the MRI apparatus 300 may receive an input for determining whether to reobtain the k-space blade having the piece of centroid data exceeding the error range. When the MRI apparatus 300 receives an input for not reobtaining the k-space blade having the piece of centroid data exceeding the error range, it may be determined that the reperformance of the image capturing is abandoned.

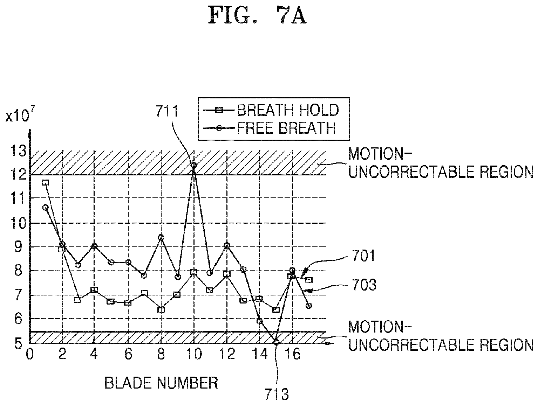

FIG. 7A is a graph displayed by the MRI apparatus 300 according to the embodiment of the present disclosure.

The MRI apparatus 300 according to the embodiment may display a graph illustrating the centroid data and illustrating whether each piece of the centroid data exceeds the error range.

Referring to FIG. 7A, the MRI apparatus 300 may simultaneously display a graph 701 regarding a state in which the object holds its breath when being imaged, and a graph 703 regarding a state in which the object does not hold its breath when being imaged.

In the graph 701 of FIG. 7A, in which the object holds its breath, the data may not be included in the motion-uncorrectable region. That is, the MRI apparatus 300 may determine that the magnitude values of the centroid data do not exceed the error range.

Some of the data in the graph 703 regarding the state in which the object does not hold its breath may be included in the motion-uncorrectable region, as shown in FIG. 7A. In detail, magnitude values 711 and 713 of the centroid data may be included in the motion-uncorrectable region. That is, the MRI apparatus 300 may determine that the magnitude values 711 and 713 of the centroid data exceed the error range. The magnitude values 711 and 713 of the centroid data may respectively correspond to a tenth blade and a fifteenth blade obtained by the MRI apparatus 300.

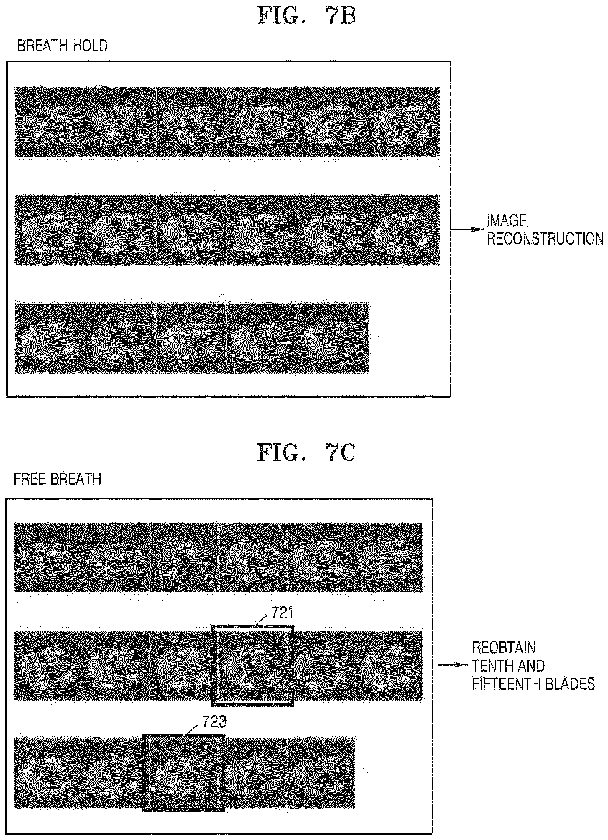

FIG. 7B shows a user interface displayed by the MRI apparatus 300 according to the embodiment of the present disclosure.

The MRI apparatus 300 may display a user interface including the MR image corresponding to each of the k-space blades.

According to the embodiment, the MRI apparatus 300 may determine that the centroid data of the obtained k-space blades does not exceed the error range. In this case, the MRI apparatus 300 may display a reconstructed image.

FIG. 7C shows another user interface displayed by the MRI apparatus 300 according to an embodiment of the present disclosure.

The MRI apparatus 300 may display a user interface including the MR image corresponding to each of the obtained k-space blades.

According to the embodiment, it may be determined that the magnitude values of the centroid data in the tenth blade and the fifteenth blade obtained by the MRI apparatus 300 exceed the error range. In this case, the MRI apparatus 300 may reobtain the tenth blade and the fifteenth blade.

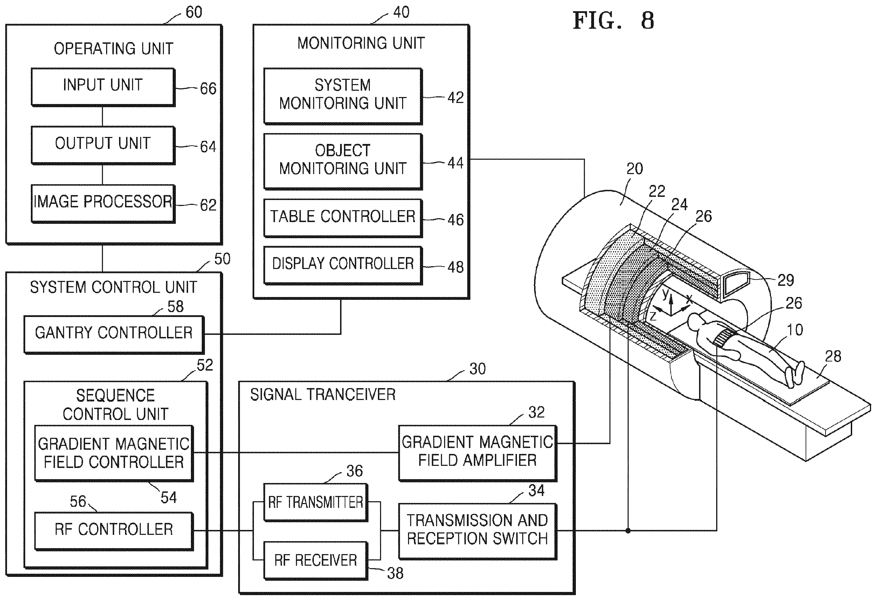

FIG. 8 is a block diagram of a general MRI system. Referring to FIG. 8, the general MRI system may include a gantry 20, a signal transceiver 30, a monitoring unit 40, a system control unit 50, and an operating unit 60.

The gantry 20 prevents external emission of electromagnetic waves generated by a main magnet 22, a gradient coil 24, and an RF coil 26. A magnetostatic field and a gradient magnetic field are formed at a bore in the gantry 20, and an RF signal is emitted toward an object 10.

The main magnet 22, the gradient coil 24, and the RF coil 26 may be arranged in a predetermined direction of the gantry 20. The predetermined direction may be a coaxial cylinder direction. The object 10 may be disposed on a table 28 that is capable of being inserted into a cylinder along a horizontal axis of the cylinder.

The main magnet 22 generates a magnetostatic field or a static magnetic field for aligning magnetic dipole moments of atomic nuclei of the object 10 in a constant direction. A precise and accurate MR image of the object 10 may be obtained due to a magnetic field generated by the main magnet 22 being strong and uniform.

The gradient coil 24 includes X, Y, and Z coils for generating gradient magnetic fields in X-, Y-, and Z-axis directions crossing each other at right angles. The gradient coil 24 may provide location information of each region of the object 10 by differently inducing resonance frequencies according to the regions of the object 10.

The RF coil 26 may emit an RF signal toward a patient and receive an MR signal emitted from the patient. In detail, the RF coil 26 may transmit, toward atomic nuclei included in the patient and having precessional motion, an RF signal having the same frequency as that of the precessional motion, stop transmitting the RF signal, and then receive an MR signal emitted from the atomic nuclei included in the patient.

For example, in order to transit an atomic nucleus from a low energy state to a high energy state, the RF coil 26 may generate and apply an electromagnetic wave signal having an RF corresponding to a type of the atomic nucleus, for example, an RF signal, to the object 10. When the electromagnetic wave signal generated by the RF coil 26 is applied to the atomic nucleus, the atomic nucleus may transit from the low energy state to the high energy state. Then, when electromagnetic waves generated by the RF coil 26 disappear, the atomic nucleus to which the electromagnetic waves were applied transits from the high energy state to the low energy state, thereby emitting electromagnetic waves having a Lamor frequency. In other words, when the applying of the electromagnetic wave signal to the atomic nucleus is stopped, an energy level of the atomic nucleus is changed from a high energy level to a low energy level, and thus the atomic nucleus may emit electromagnetic waves having a Lamor frequency. The RF coil 26 may receive electromagnetic wave signals from atomic nuclei included in the object 10.

The RF coil 26 may be realized as one RF transmitting and receiving coil having both a function of generating electromagnetic waves having an RF corresponding to a type of an atomic nucleus and a function of receiving electromagnetic waves emitted from an atomic nucleus. Alternatively, the RF coil 26 may be realized as a transmission RF coil having a function of generating electromagnetic waves having an RF corresponding to a type of an atomic nucleus, and a reception RF coil having a function of receiving electromagnetic waves emitted from an atomic nucleus.

The RF coil 26 may be fixed to the gantry 20 or may be detachable. When the RF coil 26 is detachable, the RF coil 26 may be an RF coil for a part of the object, such as a head RF coil, a chest RF coil, a leg RF coil, a neck RF coil, a shoulder RF coil, a wrist RF coil, or an ankle RF coil.

The RF coil 26 may communicate with an external apparatus via wires and/or wirelessly, and may also perform dual tune communication according to a communication frequency band.

The RF coil 26 may be a birdcage coil, a surface coil, or a transverse electromagnetic (TEM) coil according to structures.

The RF coil 26 may be a transmission exclusive coil, a reception exclusive coil, or a transmission and reception coil according to methods of transmitting and receiving an RF signal.

The RF coil 26 may be an RF coil with any one of various channels, such as 16 channels, 32 channels, 72 channels, and 144 channels.

The gantry 20 may further include a display 29 disposed outside the gantry 20 and a display (not shown) disposed inside the gantry 20. The gantry 20 may provide predetermined information to the user or the object 10 through the display 29 and the display respectively disposed outside and inside the gantry 20.

The signal transceiver 30 may control the gradient magnetic field formed inside the gantry 20, i.e., in the bore, according to a predetermined MR sequence, and control transmission and reception of an RF signal and an MR signal.

The signal transceiver 30 may include a gradient amplifier 32, a transmission and reception switch 34, an RF transmitter 36, and an RF receiver 38.

The gradient amplifier 32 drives the gradient coil 24 included in the gantry 20, and may supply a pulse signal for generating a gradient magnetic field to the gradient coil 24 under the control of a gradient magnetic field controller 54. By controlling the pulse signal supplied from the gradient amplifier 32 to the gradient coil 24, gradient magnetic fields in X-, Y-, and Z-axis directions may be synthesized.

The RF transmitter 36 and the RF receiver 38 may drive the RF coil 26. The RF transmitter 36 may supply an RF pulse in a Lamor frequency to the RF coil 26, and the RF receiver 38 may receive an MR signal received by the RF coil 26.

The transmission and reception switch 34 may adjust transmitting and receiving directions of the RF signal and the MR signal. For example, the transmission and reception switch 34 may emit the RF signal toward the object 10 through the RF coil 26 during a transmission mode, and receive the MR signal from the object 10 through the RF coil 26 during a reception mode. The transmission and reception switch 34 may be controlled by a control signal output by an RF controller 56.

Also, the signal transceiver 30 may include the MR signal receiver 310 (see FIG. 3).

The monitoring unit 40 may monitor or control the gantry 20 or devices mounted on the gantry 20. The monitoring unit 40 may include a system monitoring unit 42, an object monitoring unit 44, a table controller 46, and a display controller 48.

The system monitoring unit 42 may monitor and control a state of the magnetostatic field, a state of the gradient magnetic field, a state of the RF signal, a state of the RF coil 26, a state of the table 28, a state of a device measuring body information of the object 10, a power supply state, a state of a thermal exchanger, and a state of a compressor.

The object monitoring unit 44 monitors a state of the object 10. In detail, the object monitoring unit 44 may include a camera for observing a movement or position of the object 10, a respiration measurer for measuring the respiration of the object 10, an electrocardiogram (ECG) measurer for measuring the electrical activity of the object 10, or a temperature measurer for measuring a temperature of the object 10.

The table controller 46 controls a movement of the table 28 where the object 10 is positioned. The table controller 46 may control the movement of the table 28 according to a sequence control of a sequence controller 52. For example, during moving imaging of the object 10, the table controller 46 may continuously or discontinuously move the table 28 according to the sequence control of the sequence controller 52, and thus the object 10 may be photographed in a field of view (FOV) larger than that of the gantry 20.

The display controller 48 controls the display 29 disposed outside the gantry 20 and the display disposed inside the gantry 20. In detail, the display controller 48 may control the display 29 and the display to be on or off, and may control a screen image to be output on the display 29 and the display. Also, when a speaker is located inside or outside the gantry 20, the display controller 48 may control the speaker to be on or off, or may control sound to be output via the speaker.

The system control unit 50 may include the sequence controller 52 for controlling a sequence of signals formed in the gantry 20, and a gantry controller 58 for controlling the gantry 20 and the devices mounted on the gantry 20.

The sequence controller 52 may include the gradient magnetic field controller 54 for controlling the gradient amplifier 32, and the RF controller 56 for controlling the RF transmitter 36, the RF receiver 38, and the transmission and reception switch 34. The sequence controller 52 may control the gradient amplifier 32, the RF transmitter 36, the RF receiver 38, and the transmission and reception switch 34 according to a pulse sequence received from the operating unit 60. Here, the pulse sequence includes all information required to control the gradient amplifier 32, the RF transmitter 36, the RF receiver 38, and the transmission and reception switch 34. For example, the pulse sequence may include information for strength, an application time, and application timing of a pulse signal applied to the gradient coil 24.

The operating unit 60 may request the system control unit 50 to transmit pulse sequence information while controlling an overall operation of the MRI system.

The operating unit 60 may include an image processor 62 for receiving and processing the MR signal received by the RF receiver 38, an output unit 64, and an input unit 66.

The image processor 62 may process the MR signal received from the RF receiver 38 so as to generate MR image data of the object 10.

The image processor 62 receives the MR signal received by the RF receiver 38 and performs any one of various signal processes, such as amplification, frequency transformation, phase detection, low frequency amplification, and filtering, on the received MR signal.

The image processor 62 may arrange digital data in a k space (for example, also referred to as a Fourier space or a frequency space) of a memory, and rearrange the digital data into image data via 2D or 3D Fourier transformation.

The image processor 62 may perform a composition process or a difference calculation process on the image data if required. The composition process may be an addition process performed on a pixel or a maximum intensity projection (MIP) process performed on a pixel. The image processor 62 may store not only the rearranged image data but also image data on which a composition process or a difference calculation process is performed, in a memory (not shown) or an external server.

The image processor 62 may perform any of the signal processes on the MR signal in parallel. For example, the image processor 62 may perform a signal process on a plurality of MR signals received by a multi-channel RF coil in parallel so as to rearrange the plurality of MR signals into image data.

The image processor 62 according to the embodiment may obtain the k-space blades from the MR signal by using the PROPELLER method.

Also, the image processor 62 may obtain centroid data from the k-space blades, and may determine whether to reconstruct the MR image of the object based on the centroid data.

The output unit 64 may output image data generated or rearranged by the image processor 62 to the user. The output unit 64 may also output information required for the user to manipulate the MRI system, such as a user interface (UI), user information, or object information. The output unit 64 may be a speaker, a printer, a cathode-ray tube (CRT) display, a liquid crystal display (LCD), a plasma display panel (PDP), an organic light-emitting device (OLED) display, a field emission display (FED), a light-emitting diode (LED) display, a vacuum fluorescent display (VFD), a digital light processing (DLP) display, a flat panel display (FPD), a 3-dimensional (3D) display, a transparent display, or any one of other various output devices that are well known to one of ordinary skill in the art.

The output unit 64 according to the embodiment may also display a graph illustrating the centroid data and illustrating whether each piece of the centroid data exceeds the error range.

The output unit 64 may display a user interface including the MR images corresponding to the k-space blades.

The user may input object information, parameter information, a scan condition, a pulse sequence, or information about image composition or difference calculation by using the input unit 66. The input unit 66 may be a keyboard, a mouse, a track ball, a voice recognizer, a gesture recognizer, a touch screen, or any one of other various input devices that are well known to one of ordinary skill in the art.

The input unit 66 according to the embodiment may receive an input regarding whether to reobtain the k-space blades. Also, the input unit 66 may receive an input for determining an error range with respect to the centroid data.

The signal transceiver 30, the monitoring unit 40, the system control unit 50, and the operating unit 60 are separate components in FIG. 8, but it will be obvious to one of ordinary skill in the art that respective functions of the signal transceiver 30, the monitoring unit 40, the system control unit 50, and the operating unit 60 may be performed by another component. For example, the image processor 62 converts the MR signal received from the RF receiver 38 into a digital signal in FIG. 8, but alternatively, the conversion of the MR signal into the digital signal may be performed by the RF receiver 38 or the RF coil 26.

The gantry 20, the RF coil 26, the signal transceiver 30, the monitoring unit 40, the system control unit 50, and the operating unit 60 may be connected to each other by wire or wirelessly, and when they are connected wirelessly, the MRI system may further include an apparatus (not shown) for synchronizing clock signals therebetween. Communication between the gantry 20, the RF coil 26, the signal transceiver 30, the monitoring unit 40, the system control unit 50, and the operating unit 60 may be performed by using a high-speed digital interface, such as low voltage differential signaling (LVDS), asynchronous serial communication, such as a universal asynchronous receiver transmitter (UART), a low-delay network protocol, such as error synchronous serial communication or a controller area network (CAN), optical communication, or any of other various communication methods that are well known to one of ordinary skill in the art.

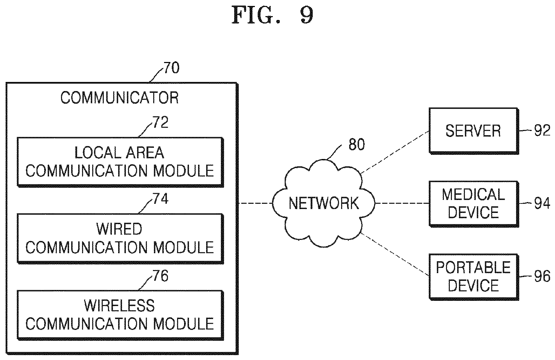

FIG. 9 is a block diagram of a communication unit 70 according to an embodiment of the present invention. Referring to FIG. 9, the communication unit 70 may be connected to at least one selected from the gantry 20, the signal transceiver 30, the monitoring unit 40, the system control unit 50, and the operating unit 60 of FIG. 8.

The communication unit 70 may transmit and receive data to and from a hospital server or another medical apparatus in a hospital, which is connected through a picture archiving and communication system (PACS), and perform data communication according to the digital imaging and communications in medicine (DICOM) standard.

As shown in FIG. 9, the communication unit 70 may be connected to a network 80 by wire or wirelessly to communicate with a server 92, a medical apparatus 94, or a portable device 96.

In detail, the communication unit 70 may transmit and receive data related to the diagnosis of an object through the network 80, and may also transmit and receive a medical image captured by the medical apparatus 94, such as a CT apparatus, an MRI apparatus, or an X-ray apparatus. In addition, the communication unit 70 may receive a diagnosis history or a treatment schedule of the object from the server 92 and use the same to diagnose the object. The communication unit 70 may perform data communication not only with the server 92 or the medical apparatus 94 in a hospital, but also with the portable device 96, such as a mobile phone, a personal digital assistant (PDA), or a laptop of a doctor or patient.

Also, the communication unit 70 may transmit information about a malfunction of the MRI system or about a medical image quality to a user through the network 80, and receive a feedback regarding the information from the user.

The communication unit 70 may include at least one component enabling communication with an external apparatus. For example, the communication unit 70 may include a local area communication module 72, a wired communication module 74, and a wireless communication module 76.

The local area communication module 72 refers to a module for performing local area communication with an apparatus within a predetermined distance. Examples of local area communication technology according to an embodiment of the present invention include, but are not limited to, a wireless local area network (LAN), Wi-Fi, Bluetooth, ZigBee, Wi-Fi direct (WFD), ultra wideband (UWB), infrared data association (IrDA), Bluetooth low energy (BLE), and near field communication (NFC).

The wired communication module 74 refers to a module for performing communication by using an electric signal or an optical signal. Examples of wired communication technology according to an embodiment of the present invention include wired communication techniques using a twisted pair cable, a coaxial cable, and an optical fiber cable, and other well known wired communication techniques.

The wireless communication module 76 transmits and receives a wireless signal to and from at least one selected from a base station, an external apparatus, and a server in a mobile communication network. Here, the wireless signal may be a voice call signal, a video call signal, or data in any one of various formats according to transmission and reception of a text/multimedia message.

The MRI apparatus 300 shown in FIG. 3 may include an external server 92, an external medical device 94, or an external portable device 96, each connected to an MRI system. That is, the MRI apparatus 300 may operate while connected to the communicator 70 shown in FIG. 9.

The embodiments of the present invention can be written as computer programs and can be implemented in general-use digital computers that execute the programs using a computer-readable recording medium.

Examples of the computer-readable recording medium include magnetic storage media (e.g., ROM, floppy disks, hard disks, etc.), optical recording media (e.g., CD-ROMs or DVDs), etc.

While the present invention has been particularly shown and described with reference to exemplary embodiments thereof, it will be understood by those of ordinary skill in the art that various changes in form and details may be made therein without departing from the spirit and scope of the present invention as defined by the following claims.

**Claims**

1. A magnetic resonance imaging (MRI) apparatus comprising:

   a magnetic resonance (MR) signal receiver configured to obtain an MR signal emitted from an object; and
   a processor configured to obtain k-space blades from the MR signal by a periodically rotated overlapping parallel lines with enhanced reconstruction (PROPELLER) method,
   to obtain centroid data of the k-space blades, and
   to determine whether to reconstruct an MR image of the object based on the centroid data.

2. The MRI apparatus of claim 1, wherein the processor is configured to reconstruct the MR image of the object based on whether there is a piece of centroid data exceeding an error range.

3. The MRI apparatus of claim 1, wherein, when there is a piece of centroid data exceeding the error range among the centroid data, the processor is configured to reobtain a k-space blade having the piece of centroid data exceeding the error range.

4. The MRI apparatus of claim 1, wherein, when there is no centroid data exceeding the error range, the processor is configured to determine that the MR image is to be reconstructed from the k-space blades.

5. The MRI apparatus of claim 1, further comprising a display configured to display a graph illustrating magnitudes of the centroid data and illustrating whether the magnitudes of the centroid data exceed the error range.

6. The MRI apparatus of claim 1, further comprising a display configured to display a user interface including MR images corresponding to the k-space blades.

7. The MRI apparatus of claim 1, further comprising an input unit configured to receive an input about whether to reobtain the k-space blades.

8. The MRI apparatus of claim 1, wherein each of the k-space blades is obtained by one scanning operation.

9. The MRI apparatus of claim 1, wherein the processor is configured to reconstruct an MR image, in which a motion is corrected, by using the k-space blades.

10. The MRI apparatus of claim 1, further comprising a display configured to display the MR image that is reconstructed.

11. A method for a magnetic resonance imaging (MRI) apparatus, the method comprising:

    obtaining a magnetic resonance (MR) signal emitted from an object;
    obtaining k-space blades from the MR signal according to a periodically rotated overlapping parallel lines with enhanced reconstruction (PROPELLER) method;
    obtaining centroid data of the k-space blades; and
    determining whether to reconstruct an MR image of the object based on the centroid data.

12. The method of claim 11, wherein the determining of whether to reconstruct the MR image comprises performing the determination based on whether there is a piece of centroid data exceeding an error range, from among the centroid data of the k-space blades.

13. The method of claim 11, further comprising, when there is a piece of centroid data exceeding the error range among the centroid data, reobtaining a k-space blade having the piece of centroid data exceeding the error range.

14. The method of claim 11, further comprising, when there is no centroid data exceeding the error range, determining that an MR image is to be reconstructed from the k-space blades.

15. The method of claim 11, further comprising displaying a graph illustrating the centroid data and illustrating whether each piece of the centroid data exceeds the error range.

16. The method of claim 11, further comprising displaying a user interface including an MR image corresponding to the k-space blades.

17. The method of claim 11, further comprising receiving an input regarding whether to reobtain the k-space blades.

18. The method of claim 11, wherein each of the k-space blades is obtained by one scanning operation.

19. The method of claim 11, further comprising reconstructing an MR image in which a motion is corrected, by using the k-space blades.

20. A non-transitory computer-readable recording medium having recorded thereon a program, which when executed by a computer, performs the method of claim 11.

FIG. 1

FIRST BLADE

$| S(k_c)_0 |$

$| S(k_c)_1 |$

$| S(k_c)_{N-1} |$

SECOND BLADE     N-TH BLADE

ky

kx

# FIG. 2

210

DETERMINE TO → IMAGE
RECONSTRUCT IMAGE    RECONSTRUCTION →

$| S(k_c)_0 |$ 내지 $| S(k_c)_{N-1} |$

MOTION CORRECTION

220

# FIG. 3

300

310

| MR SIGNAL RECEIVER | ◄──► | PROCESSOR |

320

# FIG. 4

# FIG. 5

START

OBTAIN K-SPACE BLADES — S101

OBTAIN CENTROID DATA OF
K-SPACE BLADES — S103

DETERMINE WHETHER TO RECONSTRUCT
MR IMAGE BASED ON CENTROID DATA — S105

END

# FIG. 6

```
        ( START )
            │
            ▼
┌──────────────────────────────┐
│   OBTAIN K-SPACE BLADES       │──── S201
└──────────────────────────────┘
            │
            ▼
┌──────────────────────────────┐
│   OBTAIN CENTROID DATA OF      │──── S203
│   K-SPACE BLADES              │
└──────────────────────────────┘
            │
            ▼
┌──────────────────────────────┐
│   SET ERROR RANGE             │──── S205
└──────────────────────────────┘
            │
            ▼
         ◇ S207
   IS THERE PIECE
NO OF CENTROID DATA EXCEEDING
   ERROR RANGE?
            │ YES
            ▼
┌────────────────────────────────────────┐
│ OBTAIN AGAIN K-SPACE BLADE HAVING PIECE OF │──── S209
│ CENTROID DATA EXCEEDING ERROR RANGE     │
└────────────────────────────────────────┘
            │
            ▼
┌──────────────────────────────┐
│   RECONSTRUCT IMAGE           │──── S211
└──────────────────────────────┘
            │
            ▼
         ( END )
```

# FIG. 7A

# FIG. 7B

BREATH HOLD

IMAGE
RECONSTRUCTION

# FIG. 7C

FREE BREATH

721

723

REOBTAIN
TENTH AND
FIFTEENTH BLADES

FIG. 8

EP 3 342 339 A1

# FIG. 9

INTERNATIONAL SEARCH REPORT

International application No.

**PCT/KR2016/008670**

| A. | CLASSIFICATION OF SUBJECT MATTER |
|---|---|

*A61B 5/055(2006.01)i, A61B 5/00(2006.01)i, G01R 33/54(2006.01)i, G01R 33/56(2006.01)i*

According to International Patent Classification (IPC) or to both national classification and IPC

| B. | FIELDS SEARCHED |
|---|---|

Minimum documentation searched (classification system followed by classification symbols)
A61B 5/055; G01V 3/00; G01R 33/56; G01R 33/48; G01R 33/44; A61B 5/00; G01R 33/54

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)
eKOMPASS (KIPO internal) & Keywords: magnetism, resonance, blade, error, middle point, PROPELLER

| C. | DOCUMENTS CONSIDERED TO BE RELEVANT | |
|---|---|---|
| Category* | Citation of document, with indication, where appropriate, of the relevant passages | Relevant to claim No. |
| Y | JP 2013-240713 A (KONINKL PHILIPS NV.) 05 December 2013<br>See abstract, paragraph [16] and claim 1 and figures 1-3. | 1-20 |
| Y | KR 10-2014-0066554 A (SAMSUNG ELECTRONICS CO., LTD.) 02 June 2014<br>See abstract, paragraphs [26]-[47] and claim 1. | 1-20 |
| A | US 2011-0089945 A1 (DOYLE) 21 April 2011<br>See abstract, paragraphs [37]-[41] and figures 3-5. | 1-20 |
| A | US 2005-0127912 A1 (PIPE) 16 June 2005<br>See abstract, paragraphs [35]-[39] and figures 3, 4. | 1-20 |
| A | WO 2014-165503 A1 (DIGNITY HEALTH) 09 October 2014<br>See abstract, paragraph [4] and figures 1-2B. | 1-20 |

☐ Further documents are listed in the continuation of Box C.  ☒ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 15 NOVEMBER 2016 (15.11.2016) | **16 NOVEMBER 2016 (16.11.2016)** |

| Name and mailing address of the ISA/KR | Authorized officer |
|---|---|
| Korean Intellectual Property Office<br>Government Complex-Daejeon, 189 Seonsa-ro, Daejeon 302-701,<br>Republic of Korea | |
| Facsimile No. 82-42-472-7140 | Telephone No. |

Form PCT/ISA/210 (second sheet) (January 2015)

**INTERNATIONAL SEARCH REPORT**
Information on patent family members

International application No.

**PCT/KR2016/008670**

| Patent document cited in search report | Publication date | Patent family member | Publication date |
|---|---|---|---|
| JP 2013-240713 A | 05/12/2013 | CN 101669038 A | 10/03/2010 |
| | | CN 101669038 B | 11/06/2014 |
| | | EP 2145199 A2 | 20/01/2010 |
| | | EP 2806284 A1 | 26/11/2014 |
| | | JP 2010-524622 A | 22/07/2010 |
| | | JP 2014-210209 A | 13/11/2014 |
| | | JP 5709950 B2 | 30/04/2015 |
| | | JP 5779701 B2 | 16/09/2015 |
| | | JP 5781760 B2 | 24/09/2015 |
| | | WO 2008-132659 A2 | 06/11/2008 |
| | | WO 2008-132659 A3 | 08/01/2009 |
| KR 10-2014-0066554 A | 02/06/2014 | CN 103829948 A | 04/06/2014 |
| | | CN 103829948 B | 20/04/2016 |
| | | EP 2735880 A1 | 28/05/2014 |
| | | EP 2735880 B1 | 02/09/2015 |
| | | US 2014-0145718 A1 | 29/05/2014 |
| | | US 2015-0054507 A1 | 26/02/2015 |
| | | US 8878535 B2 | 04/11/2014 |
| | | US 9244143 B2 | 26/01/2016 |
| | | WO 2014-081095 A1 | 30/05/2014 |
| US 2011-0089945 A1 | 21/04/2011 | EP 2491432 A2 | 29/08/2012 |
| | | US 8405394 B2 | 26/03/2013 |
| | | WO 2011-050078 A2 | 28/04/2011 |
| | | WO 2011-050078 A3 | 28/07/2011 |
| US 2005-0127912 A1 | 16/06/2005 | US 2005-0007114 A1 | 13/01/2005 |
| | | US 6882148 B2 | 19/04/2005 |
| | | US 7030609 B2 | 18/04/2006 |
| WO 2014-165503 A1 | 09/10/2014 | US 2016-0061924 A1 | 03/03/2016 |

Form PCT/ISA/210 (patent family annex) (January 2015)